(19) Europäisches Patentamt
European Patent Office
Office européen des brevets

(11) Publication number: **0 256 423 B1**

(12) **EUROPEAN PATENT SPECIFICATION**

(45) Date of publication of patent specification: **07.04.93** (51) Int. Cl.5: **C12P 21/02**, C12N 1/20

(21) Application number: **87111253.8**

(22) Date of filing: **04.08.87**

(54) **Strain mass-producing Epsilon-poly-L-lysine, a method for using its strain and a method for producing Epsilon-poly-L-lysine.**

(30) Priority: **19.08.86 JP 192157/86**
**19.08.86 JP 192158/86**

(43) Date of publication of application:
**24.02.88 Bulletin 88/08**

(45) Publication of the grant of the patent:
**07.04.93 Bulletin 93/14**

(84) Designated Contracting States:
**DE FR NL**

(56) References cited:

**CHEMICAL ABSTRACTS, vol. 96, no. 5, 1st February 1982, page 224, abstract no. 30265q, Columbus, Ohio, US; S. SHIMA et al.: "Poly-L-lysine produced by streptomyces. Part III. Chemical studies", & AGRIC. BIOL. CHEM. 1981, 45(1) 2503-8**

**CHEMICAL ABSTRACTS, vol. 99, no. 5, 1st August 1983, page 389, Abstract no. 4040e, Columbus, Ohio,US; S. SHIMA et al.:"Poly-L-lysine produced by streptomyces. Part V. biosynthesis of -poly-L-lysyne by washed mycelium of strep tomyces albulus No 346". NIPPON NOGEI LAGAKU KAISHI, 1983**

(73) Proprietor: **Chisso Corporation**
**6-32, Nakanoshima 3-chome Kita-ku**
**Osaka-shi Osaka-fu(JP)**

(72) Inventor: **Hiraki, Jun**
**37-3, Tomiokanishi 2-chome Kanazawa-ku**
**Yokohama-shi, Kanagawa-ken(JP)**
Inventor: **Morita, Hiroshi**
**10-1-401, Seto Kanazawa-ku**
**Yokohama-shi, Kanagawa-ken(JP)**

(74) Representative: **Kraus, Walter, Dr. et al**
**Patentanwälte Kraus, Weisert & Partner**
**Thomas-Wimmer-Ring 15**
**W-8000 München 22 (DE)**

EP 0 256 423 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

This invention relates to a strain which mass-produces ε-poly-L-lysine (abbreviated as εPL in the following), a method for using the strain and a method for producing εPL. εPL is a high-molecular weight compound as described in the following equation in which amino groups of ε-positions of L-lysine are linked to adjacent carboxyl groups of L-lysine by amide bond formation.

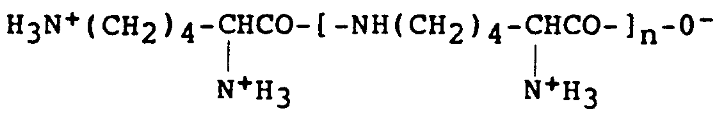

n=10-30

As this material is a polymer of L-lysine which is an essential amino-acid, it has high safety and peculiar physical properties because of its high cation content. Accordingly, its use in toiletry supplies, beauty aids, feed additives, pharmaceuticals, agricultural medicines, food additives, electronic materials, etc. is expectable, by using these properties.

Hitherto, this material was obtained by cultivating the strain of Streptomyces albulus subsp. lysinopolymerus No.346-D (deposit No.3834 of microorganisms of FRI) which is a εPL production bacterium belonging to Streptomyces in a culture medium, separating it from the obtained culture materials and refining it (Japanese Patent Publication 59-20359).

However, εPL was produced at most 0.5 g per liter of culture solution from the said strain. Accordingly, the production cost of this material was high and its wide utilization was prevented.

The inventors of the present invention have obtained a strain mass-producing εPL, investigated repeatedly in order to produce εPL in large quantities by using the said strain, and attained to the following invention.

### Summary of the Invention

The present invention provides a variant strain mass-producing εPL which is the variant FERM BP 1109 derived from Streptomyces albulus subsp. lysinopolymerus No. 346-D and which has tolerance to S-aminoethyl-L-cysteine and glycine. Further, the present invention provides a strain mass-producing εPL which is the variant FERM BP 1110 obtained by amplification treatment of a plasmid of Streptomyces albulus subsp. lysinopolymerus No. 346-D with chloramphenicol, a method for using the variant FERM BP 1109, comprising cultivating the variant FERM BP 1109 in a culture medium, storing the produced ε-poly-L-lysine in the culture solution and collecting the stored ε-poly-L-lysine from the solution and a method for using the variant FERM BP 1110, comprising cultivating the variant FERM BP 1110 in a culture medium, storing the produced ε-poly-L-lysine in the culture solution and collecting the stored ε-poly-L-lysine from the solution. The present invention also provides a method for producing εPL, characterized in that a strain which produces εPL is variant-treated, the obtained variant strain is cultivated in a culture medium to which L-lysine or L-lysine and one or more sugars are added, εPL is mass-produced and stored in the culture solution, and the stored εPL is collected from the solution.

The variant strain is a strain which produces εPL in large quantities, preferably it is a variant strain which has tolerance to an analogue of L-lysine of Streptomyces albulus subsp. lysinopolymerus No. 346-D strain or a plasmid-amplification treated strain of the same bacterium.

The analogue material of L-lysine is preferably S-aminoethyl-L-cysteine or an additive of S-aminoethyl-L-cysteine and one or more materials selected from the group of L-threonine, glycine, L-homoserine and L-methionine.

Further, the plasmid-amplification treated strain of Streptomyces albulus subsp. lysinopolymerus No.346-D strain is a strain which is obtained by amplification treatment of a plasmid, and a plasmid-amplification treated strain 50833 (deposit No.1110 of microorganisms of FRI) obtained by chloramphenicol treatment can be exemplified.

The following description serves to illustrate the invention more specifically.

Firstly, a method for obtaining the strain of the present invention is described. The variant strains which have tolerance to an L-lysine analogue such as S-aminoethyl-L-cysteine are prepared, e.g., from the following method.

Spores of Streptomyces albulus subsp. lysinopolymerus No.346-D strain are suspended in tris-maleic acid buffer solution (pH 9.0) and N-methyl-N-nitro-N'-nitrosoguanidine is added to the solution.

After shaking the solution, the spores are collected by centrifugation, washed with sterilized water and inoculated in a culture medium. By shaking the medium, bacteria are cultivated. The culture medium containing the bacteria (hereinafter the medium is referred to as culture solution) is diluted. Then, S-aminoethyl-L-cysteine or an additive of S-aminoethyl-L-cysteine and one or more materials selected from the group of glycine, L-threonine, L-homoserine and L-methionine is added to an agar culture medium having the same composition as the above medium.

In this case, S-aminoethyl-L-cysteine in concentration from 0.5 to 10 mg per ml of the agar culture medium, preferably 2mg, or an additive containing of S-aminoethyl-L-cysteine in the same concentration and one or more amino acids selected from the above group in concentration from 0.2 to 5 mg per ml of the agar culture medium, preferably 1 mg, is usable. The above diluted culture solution is applied to this agar culture medium. After incubating the applied agar culture medium, S-aminoethyl-L-cysteine variant strains are obtained as colonies. The strain cultivated in the agar culture medium containing S-aminoethyl-L-cysteine alone is a tolerance variant strain 81512. The strain cultivated in the agar culture medium containing S-aminoethyl-L-cysteine and glycine is a tolerance variant strain 11011A-1 (deposit No.1109 of microorganisms of FRI). Further, the strain cultivated in the agar culture medium containing S-aminoethyl-L-cysteine and L-threonine is a tolerance variant strain 81502.

Next, the plasmid-amplification treated strains are obtained by amplification treatment of a plasmid, e.g., by the following method. Streptomyces albulus subsp. lysinopolymerus No.346-D strains or S-aminoethytl-L-cysteine tolerance variant strain is inoculated in a culture medium. After shaking the medium, chloramphenicol is added to the medium, and cultivation is continued. Cultivated bacteria are collected by centrifugation, washed, and applied to an agar culture meduim. After static cultivation, a conventional agar culture medium containing Staphylococcus aureus is overlapped on the said medium. After further cultivation, the produced big strains of Staphylococcus aureus having boundaries for obstructing the growth are the desired plasmid-amplification treated strains which are mass-producible $\epsilon$PL, namely, plasmid-amplification treated strain 50833 (deposit No.1110 of microorganisms of FRI). Among these variant strains, the mycological properties of the 11011A-1 strain and the 50833 strain are as follows.

1. Morphological properties:

Aerical Mycel and Substrate Mycelium of the 11011A-1 strain and the 50833 strain which were grown on a sucrose-nitrate agar culture medium at 30°C for ten days were observed by a microscope. The result is as follows.

1) Ramification and morphology of the sporogenesis mycelia: simple ramification, and closed spiral.
2) Spore number: several tens.
3) Surface structure and spore size: spiny, about 1.2-1.5$\mu$, and round or oval shape.
4) Existence of flagellum spores, bacterium nucleuses and sporangia: non.
5) Insertion position of Sporophore: on Aerical Mycel.

2. Growth conditions on various culture mediums

Properties of the strains on the following various culture mediums are shown as observation results after cultivating at 30°C for 10-14 days.

a) The 11011A-1 strain (deposit No.1109 of microorganisms of FRI)

| Culture midium | Substrate Mycelium | Aerical Mycel | Soluble pigment |
|---|---|---|---|
| Sucrose-nitrate agar | pale yellow | gray brown | non |
| Glucose-asparagine agar | pale yellow | white, powdery | pale yellow |
| Glycerol-asparagine agar | pale yellow, then, yellow brown | bad growth of Aerical Mycel | pale yellow |
| Tyrosine agar | pale brown | white, rich, powdery | brown |
| Nutrient agar | pale yellow | white, rich | non |
| Yeast-maltose agar | pale yellow wrinkled | white, powdery, gray brown spots | non |
| Oatmeal agar | pale gray | white, then, gray brown | non |

b) The 50833 strain (deposit No.1110 of microorganisms of FRI)

| Culture midium | Substrate Mycelium | Aerical Mycel | Soluble pigment |
|---|---|---|---|
| Sucrose-nitrate agar | pale yellow | gray brown | non |
| Glucose-asparagine agar | pale brown | white, powdery | pale yellow |
| Glycerol-asparagine agar | pale yellow, then, yellow brown | bad growth of Aerical Mycel | pale yellow |
| Tyrosine agar | brown | white, powdery, rich | brown |
| Nutrient agar | pale yellow | white, rich | non |
| Yeast-maltose agar | pale yellow wrinkled | white, powdery, gray brown spots | non |
| Oatmeal agar | pale gray | white, then, gray brown | non |

3. Physiological properties

The physiological properties of the 11011A-1 strain and the 50833 strain are as follows.

1) Range of growth temperatures: about 15-40 ° C.

The optimum growth temperature: about 30 ° C.

2) Liquefaction of gelatine, hydrolysis of starch and peptonization of skin milk: all positive.

3) Coaguration of skin milk: negative.

4) Formation of meraninlike pigments: a brown pigment is produced on the tyrosine agar culture medium.

5) Composition of cell walls: the analytical result obtained by the method of Becker et al. (Applied Microbiology, 13, 236 (1965)) shows that the type of diaminopimelic acid in the composition of cell walls was L, L type.

4. Assimilability of various carbon sources on the Pridham Gottliep agar culture medium:

| | |
|---|---|
| L-arabinose | - |
| D-xylose | - |
| D-glucose | + |
| D-fructose | + |
| L-rhamnose | - |
| D-galactose | + |
| sucrose | - |
| raffinose | - |
| D-mannitol | + |
| i-inositol | + |
| salicin | - |
| notes: +: assimilate, -: dissimilate | |

As described above, the properties of the variant strains of the present invention are similar to the properties of Streptomyces alboulus subsp. lysinopolymerus No.346-D strain which is an original strain.

Then εPL is prepared by the present invention using the variant strains obtained by the above described methods. Further, % in this specification indicates weight(g)/volume(ml) % unless otherwise noted.

Firstly, the obtained variant strains are inoculated in a culture medium, e.g., adding L-lysine or L-lysine and one or more sugars, and cultivated. The εPL obtained from the culture medium containing the culture materials (abbreviated as culture solution hereinafter) is separated and purified. Any culture mediums containing carbon sources, nitrogen sources, inorganic salts and vitamin can be used. Although not limiting, a medium containing 5% of glucose or 5% of glycerin as the carbon source, and ammonium sulfate, L-lysine or peptone as the nitrogen source is preferred. In the course of the cultivation, the carbon source and the nitrogen source can be added successively or continuously to the culture medium.

When L-lysine and sugars are added to the culture solution, L-lysine and sugars can be added anytime from the beginning to the ending of the cultivation, preferably in the middle of the cultivation when pH is lowering. As L-lysine to be added, L-lysine monohydrochloride can be used in the range of 0.05-2% based on the total volume of the culture solution, preferably 0.5%. For the sugars, one or more sugars selected from the group of glucose, sucrose, maltose, starch, galactose, etc., and glycerine can be used in the range of 0.5-5% based on the total volume of the culture solution, preferably 2.5% of glucose can be used.

In succesive addition, L-lysine and sugars are added when the sugar concentration in the culture solution is lowered below a fixed %. For example, 2.5% of glucose and 0.5% of L-lysine are preferably added when the sugar concentration is lowered below 0.1%.

In continuous addition, a glucose solution and an L-lysine solution can be passed through a culture tank and the culture solution can be eliminated so as to maintain, for example, the glucose concentration, e.g., at 1% and, for example, L-lysine concentration, e.g., at 0.2% in the culture solution. Further, a defoaming agent can be added to the culture solution.

The pH value can be left to drop up to 4.0 at the beginning of the cultivation, and then maintained at 4.0 by adding an alkali solution such as an aqueous sodium hydroxide solution. After removing the cultivated bacteria with a centrifuge or a filter from the culture solution, the filtrate is purified, decolorized and concentrated. εPL is crystallized out of the concentrated solution by using an organic solvent such as acetone, ethanol, etc..

The effects of the present invention are as follows.

The variant strains of the present invention have the ability to mass-produce εPL. By cultivating the said variant strains, the strains produce more εPL than the known strains. Further, according to the present invention, when the variant strains of the strains producing εPL are cultivating, the strains can produce εPL in large quantities by adding L-lysine or L-lysine and one or more sugars to the culture solution.

Accordingly, the production cost of εPL can be greatly lowered in comparison with the conventional methods.

## Description of the Preferred Embodiments

The following non-limiting examples illustrate the present invention more specifically.

Example 1

Obtention of variant strains tolerant to S-aminoethyl-L-cysteine:

Spores of Streptomyces albulus subsp. lysinopolymerus No.346-D strain in quantities of a platinum earpick were suspended in tris-maleic acid buffer solution (pH 9.0) and N-methyl-N-nitro-N′-nitrosoguanidine was added to the solution. After shaking the solution at 30 ° C for 30 minutes, the spores were collected with a centrifuge, washed with sterilized water and inoculated in 5 ml of the culture medium of pH 6.8 that contains 5% of glucose, 1% of ammonium sulfate, 0.5% of yeast extract, 0.136% of potassium dihydrogen phosphate heptahydrate, 0.158% of disodium hydrogen phosphate dodecahydrate, 0.05% of magnesium sulfate heptahydrate, 0.004% of zinc sulfate heptahydrate, 0.003% of ferrous sulfate heptahydrate (the medium is abbreviated as the first culture medium hereinafter). By shaking cultivation at 30 ° C for a whole day and night, bacteria were grown.

The culture solution was diluted 5000-fold by adding the MS solution which contains of 0.05% of magnesium sulfate heptahydrate, 0.5% of sodium chloride and 0.05% of Tween 80 (Trade mark). Then, the diluted culture solution was applied to the agar culture medium having the same component as that of the first culture medium in addition to S-aminoethyl-L-cysteine or S-aminoethyl-L-cysteine in concentration of 2 mg per ml of the agar culture medium and glycine or L-threonine in concentration of 1 mg per ml of the agar culture medium. The applied agar culture medium was incubated at 30 ° C for 48 hours to grow strains as colonies, and variant strains tolerant to S-aminoethyl-L-cysteine were obtained.

In this case, the strain obtained from the agar culture medium to which only S-aminoethyl-L-cysteine was added, was the 81512 strain. One of the strains obtained from the agar culture medium to which S-aminoethyl-L-cysteine and glycine were added, was the 11011A-1 strain (deposit No.1109 of microorganisms of FRI). The strain obtained from the agar culture medium to which S-aminoethyl-L-cysteine and L-threonine were added, was the 81502 strain.

Production of εPL:

S-Aminoethyl-L-cysteine tolerance 81512 strains in quantities of a platinum earpick were inoculated in 5 ml of the culture medium having the same composition as that of the above first cluture medium, cultivated with shaking at 30 ° C for eight days. At the conclusion of the cultivation, the concentration of εPL in the culture solution was determined by the method of Itzhaki.

The yield of εPL per liter of the culture solution was 0.67 gr.

Examples 2 and 3

Using the same method as used in Example 1 except that the variant strain 81512 tolerant to S-aminoethyl-L-cysteine was changed to the variant strain 11011A-1 tolerant to S-aminoethyl-L-cysteine and glycine (deposit No.1109 of microorganisms of FRI) (Example 2) and the variant strain 81502 tolerant to S-aminoethyl-L-cysteine and L-threonine (Example 3), εPL was produced and its concentration was determined by the same method as in Example 1.

The yields of εPL per liter of the culture solution were 0.88 gr.(Example 2) and 0.72 gr.(Example 3), respectively.

Example 4

Obtention of the plasmid-amplification treated strains:

The variant strain tolerant to S-aminoethyl-L-cysteine that was obtained in Example 1 was inoculated in 5 ml of the culture medium having the same composition as that of the first culture medium as described in Example 1.

After shaking the culture medium at 30 ° C for two days, chloramphenicol in quantities from 50 to 500 mg per litre of the culture solution, preferably 100 mg, was added to the solution, and the cultivation was continued for further 5-10 hours, preferably eight hours. The cultivated bacteria were collected by centrifugation, washed with sterilized water or a physiological saline solution and applied to an agar culture medium having the same composition as that of the first culture medium in addition to 1.7% of agar.

After static cultivation at 30 ° C for 8 days, a conventional agar culture medium containing Staphylococcus aureus was overlapped on the said medium. After cultivating further one night, the produced big strains

of staphylococcus aureus having boundaries for obstructing the growth were the plasmid-amplification treated strains which could largely produce εPL. One of these strains was the 50833 strain (deposit No.1110 of microorganisms of FRI).

Production of εPL:

Using the same method as described in Example 1 except that the 81512 strain was changed to the obtained plasmid-amplification treated 50833 strain, εPL was produced and its concentrarion was determined by the same method as in Example 1.

The yield of εPL per liter of the culture solution was 1.80 gr.

Comparison Example 1

Using the same method as described in Example 1 except that strain of the variant strain 81512 tolerant to S-aminoethyl-L-cysteine was changed to Streptomyces albulus subsp. lysinopolymerus No.346-D strain, εPL was produced and its concentration was determined by the same method as in Example 1.

The yield of εPL per liter of the culture solution was 0.20 gr.

Example 5

Using the same method as described in Example 4 except that the production of εPL was conducted as follows.

The plasmid-amplification treated strains 50833 in quantities of a platinum earpick were inoculated in 5 ml of the culture medium having the same composition as that of the above cluture medium in addition to 0.5% of L-lysine monohydrochloride, cultivated with shaking at 30 °C for eight days. At the conclusion of the cultivation, the concentration of εPL in the culture solution was determined by the method of Itzhaki.

The yield of εPL per liter of the culture solution was 1.85 gr.

Comparison Example 2

Using the same method as described in Example 5 except that the plasmid-amplification treated strain 50833 was changed to Streptomyces albulus subsp. lysinopolymerus No.346-D strain, εPL was produced and its concentration was determined by the same method as in Example 5.

The yield of εPL per liter of the culture solution was 0.16 gr.

Example 6

To 1.5 liter of the culture medium containing the same component as that of the first culture medium described in Example 1, 0.05 volume % of an defoaming agent of a polyoxyalkylene glycol derivative was added. The culture solution 50 ml in which the variant strains 11011A-1 tolerant to S-aminoethyl-L-cysteine were precultivated was inoculated in the said medium, and the strains were cultivated with shaking of 600 rpm at 30 °C in an air flow rate of 2 l/min..

After 24 hours, 5% of glucose and 1% of ammonium sulfate were sterilely added to the medium. After lowering of the pH, 6N of sodium hydroxide was added while the pH was automatically and continuously controlled with a pH controller so as not to lower below 4.0. After the cultivation, the bacteria were removed with a centrifuge and εPL in the culture solution was purified with an anion exchange resin of IRA-402, a cation exchange resin of IRC-50 and active carbon of Carboraffin 50w, and the purity of the obtained εPL was 99.9 weight % and the yield of εPL per liter of the culture solution was 4.77 gr.

Comparison Example 3

Using the same method as described in Example 6 except that the variant strain 11011A-1 tolerant to S-aminoethyl-L-cysteine was changed to Streptomyces albulus subsp. lysinopolymerus No.346-D strain, εPL was produced. The purity of the obtained εPL was 97.8 weight % and the yield of εPL per liter of the culture solution was 0.56 gr.

Example 7

To 1.5 liter of the culture medium containing the same component as that of the first culture medium described in Example 1, 0.05 volume % of a defoaming agent of a polyoxyalkylene glycol derivative was added. 50 ml of the culture solution in which the plasmid-amplification treated strains 50833 were precultivated was inoculated in the said medium, and the strains were cultivated with aeration and stirring at 30°C for eight days. When the pH of the culture solution began to lower, 2.5% of glucose and 0.5% of L-lysine monohydrochloride were sterilely added to the medium. Thereafter, so as not to lower the glucose concentration of the culture solution below 2%, 2.5% of glucose was sterilily added successively. After lowering the pH, 6N of sodium hydroxide was added while the pH was automatically and continuously controlled with a pH controller so as not to lower below 4.0.

After the cultivation, the bacteria were removed with a centrifuge and the concentration of εPL in the culture solution was determined by the method Itzhaki. The yield of εPL per liter of the culture solution was 20.3 gr.

Comparison Example 4

Using the same method as described in Example 7 except that the plasmid-amplification treated strain 50833 was changed to Streptomyces albulus subsp. lysinopolymerus No.346-D strain, the concentration of εPL was determined by the same method as in Example 7.

The yield of εPL per liter of the culture solution was 0.20 gr.

Example 8

To 1.5 liter of the culture medium containing the same component as that of the first culture medium described in Example 1, 0.05 volume % of a defoaming agent of a polyoxyalkylene glycol derivative was added. 50 ml of the culture solution in which the plasmid-amplification treated strains 50833 were precultivated was inoculated in the said medium, and the strains were cultivated with shaking of 600 rpm at 30°C in an air flow rate of 2 l/min..

After 24 hours, as the pH of the culture solution began to lower, a glucose solution and a L-lysine solution were passed through the culture tank so as to maintain the concentrations of 1% of gulucose and 0.2% of L-lysine, and exhausted the culture solution. After the pH was lowered, 6N of sodium hydroxide was added while the pH was automatically and continuously controlled with a pH controller so as not to lower below 4.0.

After the cultivation, the bacteria were removed with a centrifuge, and εPL in the culture solution was purified with an anion exchange resin of IRA-402, a cation exchange resin of IRC-50 and active carbon of Carboraffin 50w. Then εPL was crystallized with alcohol. The purity of the obtained εPL was 99.9 weight % and the yield was 5.02 gr.

## Claims

1. A strain mass-producing ε-poly-L-lysine which is the variant FERM BP 1109 derived from Streptomyces albulus subsp. lysinopolymerus No. 346-D and which has tolerance to S-aminoethyl-L-cysteine and glycine.

2. A strain mass-producing ε-poly-L-lysine which is the variant FERM BP 1110 obtained by amplification treatment of a plasmid of Streptomyces albulus subsp. lysinopolymerus No. 346-D with chloramphenicol.

3. A method for using the variant FERM BP 1109 mass-producing ε-poly-L-lysine, comprising cultivating the variant FERM BP 1109 in a culture medium, storing the produced ε-poly-L-lysine in the culture solution and collecting the stored ε-poly-L-lysine from the solution.

4. A method for using the variant FERM BP 1110 mass-producing ε-poly-L-lysine, comprising cultivating the variant FERM BP 1110 in a culture medium, storing the produced ε-poly-L-lysine in the culture solution and collecting the stored ε-poly-L-lysine from the solution.

5. A method for producing ε-poly-L-lysine, comprising cultivating the variant FERM BP 1110 in a culture medium to which L-lysine and one or more sugars are added, storing the mass-produced ε-poly-L-lysine in the culture solution and collecting the stored ε-poly-L-lysine from the solution.

6. A method as claimed in claim 5, wherein the addition of L-lysine and sugars to the culture medium is conducted successively.

7. A method as claimed in claim 6, wherein the addition of L-lysine and sugars to the culture medium is conducted continuously.

**Patentansprüche**

1. Stamm zur Massenproduktion von ε-Poly-L-lysin, dadurch **gekennzeichnet,** daß er die Variante FERM BP 1109, abgeleitet von Streptomyces albulus subsp. lysinopolymerus Nr. 346-D, ist, und daß er gegenüber S-Aminoethyl-L-cysteinund Glycin tolerant ist.

2. Stamm zur Massenproduktion von ε-Poly-L-lysin, dadurch **gekennzeichnet,** daß er die Variante FERM BP 1110 ist, erhalten durch Amplifikationsbehandlung eines Plasmids von Streptomyces albulus subsp. lysinopolymerus Nr. 346-D mit Chloramphenicol.

3. Verfahren zur Verwendung der Variante FERM BP 1109, welche ε-Poly-L-lysin in Massen produziert, dadurch **gekennzeichnet,** daß man die Variante FERM BP 1109 in einem Kulturmedium züchtet, das so erzeugte ε-Poly-L-lysin in der Kulturlösung speichert und das gespeicherte ε-Poly-L-lysin aus der Lösung gewinnt.

4. Verfahren zur Verwendung der Variante FERM BP 1110, die ε-Poly-L-lysin in Massen produziert, dadurch **gekennzeichnet,** daß man die Variante FERM BP 1110 in einem Kulturmedium züchtet, das so erzeugte ε-Poly-L-lysin in der Kulturlösung speichert und das gespeicherte ε-Poly-L-lysin aus der Lösung gewinnt.

5. Verfahren zur Erzeugung von ε-Poly-L-lysin, dadurch **gekennzeichnet,** daß man die Variante FERM BP 1110 in einem Kulturmedium, dem L-Lysin und ein oder mehrere Zucker zugesetzt sind, züchtet, das in Massen erzeugte ε-Poly-L-lysin in der Kulturlösung speichert und das gespeicherte ε-Poly-L-lysin aus der Lösung gewinnt.

6. Verfahren nach Anspruch 5, dadurch **gekennzeichnet,** daß die Zugabe von L-Lysin und Zuckern zu dem Kulturmedium sukzessive durchgeführt wird.

7. Verfahren nach Anspruch 6, dadurch **gekennzeichnet,** daß die Zugabe von L-Lysin und Zuckern zu dem Kulturmedium kontinuierlich durchgeführt wird.

**Revendications**

1. Souche pour la production en masse de ε-poly-L-lysine, qui est le variant FERM BP 1109 dérivé de Streptomyces albulus sous-espèce lysinopolymerus No. 346-D et qui présente une tolérance pour la S-aminoéthyl-L-cystéine et la glycine.

2. Souche pour la production en masse de ε-poly-L-lysine, qui est le variant FERM BP 1110 obtenu par traitement d'amplification d'un plasmide de Streptomyces albulus sous-espèce lysinopolymerus No. 346-D avec du chloramphénicol.

3. Méthode pour utiliser le variant FERM BP 1109 pour la production en masse de ε-poly-L-lysine, comprenant la culture du variant FERM BP 1109 dans un milieu de culture, le stockage de la ε-poly-L-lysine produite dans la solution de culture et la récupération de la ε-poly-L-lysine stockée à partir de la solution.

4. Méthode pour utiliser le variant FERM BP 1110 pour la production en masse de ε-poly-L-lysine, comprenant la culture du variant FERM BP 1110 dans un milieu de culture, le stockage de la ε-poly-L-lysine produite dans la solution de culture et la récupération de la ε-poly-L-lysine stockée à partir de la solution.

5. Méthode pour produire de la ε-poly-L-lysine, comprenant la culture du variant FERM BP 1110 dans un milieu de culture auquel sont ajoutés de la L-lysine et un ou plusieurs sucres, le stockage de la ε-poly-L-lysine produite en masse dans la solution de culture et la récupération de la ε-poly-L-lysine stockée à partir de la solution.

6. Méthode suivant la revendication 5, dans laquelle l'addition de L-lysine et de sucres au milieu de culture est réalisée de façon successive.

7. Méthode suivant la revendication 5, dans laquelle l'addition de L-lysine et de sucres au milieu de culture est réalisée en continu.